# EUROPEAN PATENT APPLICATION

(11) **EP 0 647 712 A1**
(43) Date of publication of application: **12.04.1995**
(21) Application number: 93116221.8
(22) Date of filing: 07.10.1993
(51) Int. Cl.: C12N 15/54, C12P 19/40, C12N 15/70, C12N 1/21

(54) **Production of S-adenosyl-methionine (SAM) by fermentation of transformed bacteria**

(71) Applicant: BOEHRINGER INGELHEIM ESPANA S.A., E-08080 Barcelona (ES)
(72) Inventor: Mato, José Maria, E-28230 Las Rozas (Madrid) (ES); Pajares, Maria Angeles, E-28020 Madrid (ES); Mingorance, Jesus, E-28320-Pinto (Madrid) (ES); Avarez, Luis, ES-28035 Madrid (ES)
(74) Representative: Laudien, Dieter

(57) **Abstract**

The present invention relates to a method of production of S-adenosyl-L-methionine (SAM) by fermentation of bacteria, especially bacteria transformed with an expression vector for SAM synthetase, expression vectors which can be used for said production, bacterial strains transformed with said vectors, and a method of producing said strains.

SEQUENCE LISTING

(1) GENERAL INFORMATION:

(i) APPLICANT:
(A) NAME: Boehringer Ingelheim Espana, S.A.
(B) STREET: Apartado 14278
(C) CITY: Barcelona
(E) COUNTRY: Spain/Espana
(F) POSTAL CODE (ZIP): 08080
(G) TELEPHONE: (93)4045100
(H) TELEFAX: (93)2042850
(I) TELEX: 54722

(ii) TITLE OF INVENTION: Production of S-Adenosyl-methionine (SAM) by
fermentation of transformed bacteria

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 1:

CGGAATCCAT ATGAATGGAC CTGTGG 26

(2) INFORMATION FOR SEQ ID NO: 2:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 2:

GCACTCGAGG CTCTAAAACA CAAG 24

## Description

The present invention relates to a method of production of S-adenosyl-L-methionine (SAM) by fermentation of bacteria, especially bacteria transformed with an expression vector for SAM synthetase, expression vectors which can be used for said production, bacterial strains transformed with said vectors, and a method of producing said strains.

SAM is a naturally occurring molecule, widely distributed throughout body tissues and fluids. It plays a central role in three main metabolic pathways, such as transmethylation, transsulphuration and aminopropylation. Through methylation, SAM regulates liver membrane lipid composition and fluidity, whereas by activating the transsulphuration pathway, it favors endogenous detoxifying processes.

The enzymatic machinery required for the synthesis of SAM is ubiquitous and occurs in almost all cells. SAM is synthesized from ATP and methionine, a reaction catalyzed by the enzyme S-adenosylmethionine synthetase (SAM synthetase, EC 2.5.1.6, Cantano, G. L. J. Biol. Chem. 1953, 204: 403-416).

SAM ist currently used in medical and pharmacological areas due to its therapeutic potential in liver damage and affective disorders. A variety of clinical and experimental studies has demonstrated the ability of SAM to prevent or ameliorate the hepatotoxic effects of several drugs and chemicals (Friedel H. A. *et al.,* Drugs 1989, 38: 389-416; Corrales *et al.,* Drug Invest. 1992, 4, Suppl. 4: 8-13), to treat intrahepatic cholestasis of pregnancy (Frezza, M. *et al.,* Hepatogastroenterology 1990, 37: 122-125) and to improve clinical symptoms in cholestatic patients with chronic liver disease (Frezza, M. *et al.,* Hepatology 1987, 7: 1105; Frezza, M. *et al.,* Gastroenterology 1990, 99: 211-215). SAM has also been successfully used as an antidepressant (Baldessarini, R. J. *et al.,* Am. J. Med. 1987, 83 suppl. 5A: 95-103; Bell, K. M. *et al.,* Am. J. Psychiat. 1988, 145: 1110-1114; Chawla, R. K. *et al.,* Drugs 1990, 40 suppl. 3: 98-110). Increased therapeutic use of SAM has been made possible by the availability of a stable salt. However, the efficiency of the current methods of obtaining SAM is still low, despite attempts to produce it by enzymatic methods and/or conventional fermentation (Gross, A. *et al.,* Appl. Biochem. Biotechnol. 1983, 8: 415-422; Shiozaki, S. *et al.,* Agric. Biol. Chem. 1984, 48: 2293). Shiomi *et al.* described the use of selected yeast strains which accumulated SAM for the production of the compound (Biotechnology & Bioengineering 1990, 35: 1120-1124).

The aim of the present invention is to provide an improved method of production of SAM as well as to provide means for performing this method. For this purpose, a bacterial strain suitable for producing high levels of SAM has been prepared. The process of preparation of this bacterial strain comprises the construction of a recombinant plasmid able to express rat liver SAM synthetase at high levels, its introduction into bacterial cells, growth of cells, induction of expression and isolation of SAM in a conventional manner.

The inducible prokaryotic expression vector used was pT7-7 (Tabor, S. & Richardson, C. C., Proc. Natl. Acad. Sci. USA 1985, 82: 1074-1078). This plasmid is available from Stan Tabor (Harvard Medical School, Boston, Ma). It contains a T7 RNA promoter upstream of the polycloning site, so that expression of the cloned fragment is directed by T7 RNA polymerase.

The bacterial strain used for transformation was *E. coli* BL21 (DE3) (F-ompT r⁻_{B} m⁻_{B}) (Studier, W. F. *et al.,* Methods Enzymol. 1990, 185: 60-89), which is available from William F. Studier (Brookhaven National Laboratory). This strain carries the gene for T7 RNA polymerase, which is expressed under the control of the *lac*UV5 promoter. This promoter is inducible by isopropyl-β-D-thiogalactopyranoside (IPTG). Thus, the addition of IPTG induces the expression of T7 RNA polymerase, which, in turn, drives the expression of the cloned fragment in the plasmid pT7-7.

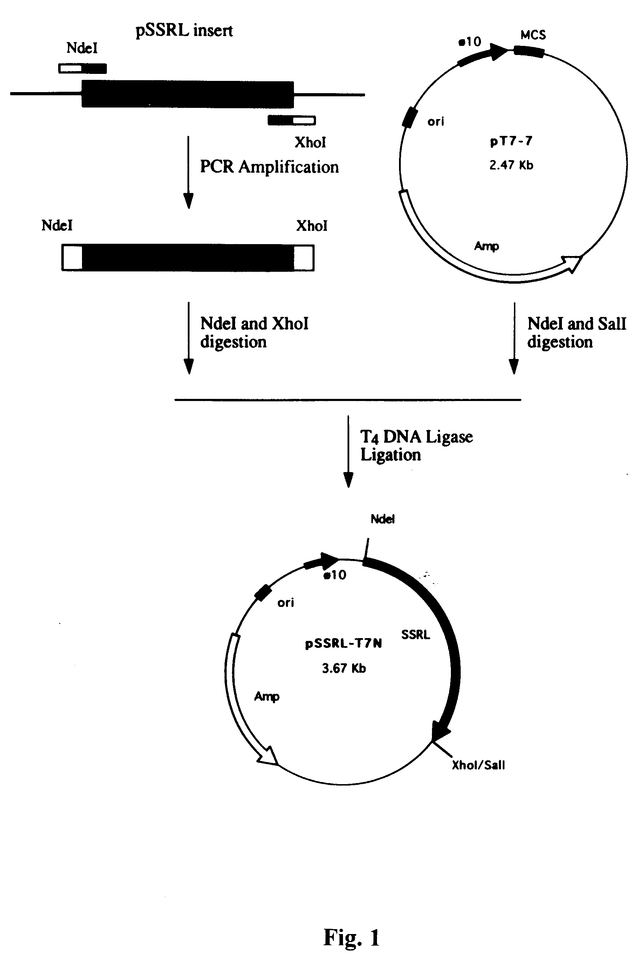

For constructing the recombinant plasmid, a 1.2 kb sequence of the cDNA clone pSSRL (Alvarez, L. *et al.,* FEBS Lett. 1991, 290: 142-146), corresponding to the coding region of rat liver SAM synthetase, was adapted for cloning into the *Nde*I/*Sal*I sites of the expression vector pT7-7 by means of the Polymerase Chain Reaction (PCR; Saiki, R. K. *et al.,* Science 1985, 230: 1350-1354). The resulting construct was designated pSSRL-T7N (Figure 1). The recombinant plasmid was introduced into *E. coli* BL21 (DE3) cells by the CaCl₂ method (Dagert, M. & Erlich, S. D., Gene 1974, 6: 23-28) and deposited under the accession number DSM 8592.

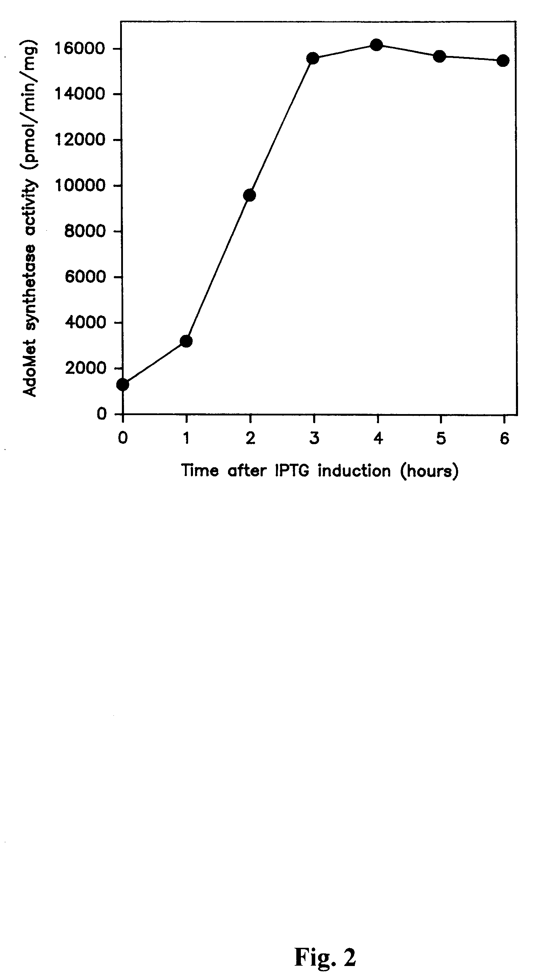

For monitoring SAM synthetase production, enzyme activity was assayed at different times after IPTG induction. Highest levels of SAM synthetase activity were obtained three hours after induction of the enzyme expression with IPTG (Figure 2). Consequently, the measurements of SAM levels were carried out three hours after the induction in either bacterial cells bearing pSSRL-TN7 or in the original strain. Non transformed *E. coli* BL21 (DE3) strain yielded an average of 0.1 nmol of SAM per ml of culture (1,2 nmol/mg protein), while the transformed strain yielded an average of 28 nmol of SAM per ml of culture (480 nmol/mg protein). Thus, a 300-fold increase of SAM content is observed in transformed cells, as compared with the original strain (Table 1). SAM can be isolated from the culture by known methods (Schlenk, F. *et* De Palma, R. E., J. Biol. Chem. 1957, 229: 1037-50; Cantoni, G. L., Methods Enzymol. 1957, 3: 600-3; Schlenk, F. *et al.,* Arch. Biochem. Biophys. 1959, 83: 28-34; Svikla, G. *et* Schlenk, F., J. Bacteriol. 1960, 79: 841; Gordon, R. K. *et al.,* Methods Enzymol. 1987, 143: 191-5; Shiomi *et al., loc. cit.*)

The method of the present invention has superior properties with respect to prior approaches to SAM production by fermentation of a microorganism. Bacterial strains obtainable as described in this specification therefore have a potential industrial interest as a powerful source of SAM.

**Table 1**

| SAM content in transformed with pSSRL-T7N and nontransformed *E. coli* BL21 (DE3). | | |
|---|---|---|
| Strain | nmol SAM/ml culture | nmol SAM/mg protein |
| *E. coli* BL21 (DE3) | 0.12 ± 0.04 | 1.1 ± 0.5 (3)^{a} |
| *E. coli* BL21 (DE3)/pSSRL-T7N | 28.6 ± 6.4 | 482 ± 30 (3) |

| | | |
|---|---|---|
| ^{a}Number of determinations | | |

### Figure legends

- **Fig. 1:**: Construction of the expression plasmid pSSRL-T7N.
A PCR fragment containing the coding sequence of rat liver SAM synthetase was digested by *Nde*I and *Xho*I restriction enzymes The resulting fragment was then ligated to pT7-7 to obtain the recombinant plasmid pSSRL-T7N.
- **Fig. 2:**: SAM synthetase activity of *E. coli* BL21 (DE3) cells transformed with pSSRL-T7N, measured at different times after IPTG induction.

### Examples

### 1. Construction of pSSRL-T7N

Two primers (5'CGGAATCCATATGAATGGACCTGTGG 3') and (3'GAACACAAAATC TCGGAGCTCACG5') were synthesized with an Applied Biosystem 391 DNA synthesizer. The first one consisted of a sequence containing *Eco*RI and *Nde*I restriction sites at the 5'-end and a 16-nucleotide sequence at the 3'-end homologous to the bases 1-16 of the rat liver SAM synthetase clone pSSRL (Alvarez, L. *et al.,* FEBS Lett. 1991, 290: 142-146). The second one had a *Xho*I site at the 5'-end and a 15-nucleotide sequence complementary to the bases 1183-1197 of the pSSRL clone. The PCR was carried out in a reaction mixture containing 150 ng of pSSRL plasmid, 25 pmol each of the two oligonucleotides, 0.04 mM each of the four deoxyribonucleotide, 2 mM MgCl₂, 50 mM KCl, 10 mM Tris-HCl pH 9.0, 0.1% Triton 100X and 2.5 units of Taq DNA polymerase (Promega Inc., Madison, Wi) in a total volume of 50 µl of mineral oil (Sigma Chemical Co., St. Louis, Mo). Amplification was performed for 30 cycles at 94°C for 2 min., 55°C for 1 min., and 72°C for 3 min, in a thermal cycler (Gene ATAQ controller, Pharmacia LKB). Thus, the amplified product contained the rat liver SAM synthetase coding region with a *Nde*I restriction site and *Xho*I site attached to the 5'- and 3'-ends of the cDNA, respectively. This product was then purified by phenol-chloroform extraction, digested with NdeI and *Xho*I and ligated to the *Nde*I-*Sal*I sites of the expression vector pT7-7, using T4 DNA ligase (Biotech SA, Madrid).

The ligation mixture was used to transform *E. coli* DH5α (relevant genotype: Δ*lac*U169, *hsd*R17, *rec*A1) using the method of CaCl₂ (Dagert, M. & Erlich, S. D., Gene 1974, 6: 23-28). Transformants were screened for plasmids containing the cDNA insert following digestion with *Nde*I and *Xho*I. One of these plasmids designated as pSSRL-T7N was then used to transform *E. coli* BL21 (DE3) cells. The resulting strain was deposited at the German Collection of Microorganisms and Cell Cultures (DSM), Mascheroder Weg 1b, D-38124 Braunsschweig, Germany, under the accession number DSM 8592.

### 2. Culture of transformed BL21(DE3) and induction of SAM synthetase expression

An overnight culture was prepared from BL21(DE3) cells bearing the recombinant plasmid pSSRL-T7N. 1 ml of this culture was used to inoculate 100 ml LB medium (10 g bactotryptone, 5 g yeast extract, 10 g NaCl per liter) containing 100 ml/mg ampicillin. The cells were grown to A₅₉₅ 0.3-0.4 and isopropyl-β-D thiogalactopyranoside (IPTG) was added to a final concentration of 0.5 mM. Aliquots of 1 ml of the culture were harvested by centrifugation at 1,2 and 3 hours after the initiation of induction. The pellets obtained were washed in water and resuspended in 100 µl of 50 mM Tris pH 8.0, 1 mM EDTA, 10 mM MgSO₄ and protease inhibitors (2 µg/ml aprotinin, 1 µg/ml pepstatin A, 0.5 µg/ml leupeptine, 2.5 µg/ml antipain, 0.1 mM benzamidine, 0.1 mM PMSF) and sonicated. After centrifugation, the supernatants were used for measuring SAM synthetase activity.

### 3. Determination of SAM synthetase activity

SAM synthetase activity was assayed has described by Cabrero *et al.* (Eur. J. Biochem. 1987, 170: 299-304) using 160 µl fraction samples and 90 µl of a reaction mixture containing 75 mM Tris/HCl pH 8.0, 250 mM KCl, 9 mM MgCl₂, 10 mM dithiothreitol, 5 mM methionine and 5 mM [2-³H]ATP (4 Ci/mol). The incubation was carried out for 30 min at 37°C and was stopped by the addition of 3 ml distilled water. The solutions were immediately loaded on cation exchanger AG 50W-X4 columns (1 ml) equilibrated in water. The columns were washed with water (20 ml) and the [³H]SAM formed was then eluted with 4 ml 3 vM ammonium hydroxide. The radioactivity was determined by counting in 10 ml of scintilliation liquid in the presence of 1 ml glacial acetic acid.

Protein was determined by the method of Bradford (Bradford M. M., Anal. Biochem. 1977, 252: 248-254) using BSA as standard.

### 4. Measurement of SAM levels

SAM levels were determined in either bacterial cells bearing pSSRL-TN7 or non-transformed cells. Three hours alter IPTG induction, aliquots of 1 ml of culture were immediately deproteinized by homogenization in 10% Trichloroacetic acid dissolved in 0.05 N HCl. TCA was eliminated by three successive washes with diethyl ether saturated with 0.05 N HCl, and samples were lyophilized. Samples were then resuspended in 0.01 M ammonium formiate pH 4.0 and analyzed in a ultrasil CX HPLC column (4.6 mm x 25 cm; particle size 10 µm) (Beckman, Palo Alto, Ca). After a 5 min washing period with 0.01 M ammonium formiate pH 4,0, SAM was eluted in a 50 min gradient from 0.01 M to 0.8 M ammonium formiate pH 4.0 at a flow rate of 1 ml/min. SAM was detected by measuring absorbance at 254 nm.

For measuring protein concentration, aliquots of 1 ml of the same cultures were centrifuged, resuspended in 100 µl of 50 mM Tris pH 8.0, 1 mM EDTA, 10 mM MgSO₄, and sonicated. Protein was determined by the method of Bradford (Bradford M. M., Anal. Biochem. 1977, 252: 248-254) using BSA as standard.

## Claims

1. Method of production of S-adenosyl-methionine by fermentation of a microbial host, characterized in that said host is a bacterium.

2. Method of claim 1, wherein said bacterium is overproducing S-adenosyl-methionine synthetase.

3. Method of claim 2, wherein said bacterium is transformed with an expression vector, said expression vector containing a nucleic acid sequence coding for S-adenosyl-methionine synthetase.

4. Method of claim 2, wherein said nucleic acid sequence is functionally linked to a bacteriophage T7 promotor.

5. Method of claims 1 to 4, wherein said bacterium is *Escherichia coli.*

6. Method of claim 5, wherein said *Escherichia coli* expresses bacteriophage T7 polymerase.

7. Method of claim 6, wherein said bacteriophage T7 polymerase expression is inducible.

8. Method of claims 2 to 7, wherein said S-adenosyl-methionine synthetase is from the rat.

9. Method of claims 5 to 8, wherein said *Escherichia coli* is the *Escherichia coli* strain deposited as DSM 8592.

10. Vector for expression of a gene in a bacterium, characterized in that it contains a nucleic acid sequence coding for S-adenosyl-methionine synthetase.

11. Vector of claim 10, wherein said nucleic acid sequence is functionally linked to a bacteriophage T7 promotor.

12. Vector of claims 10 to 11, which is a plasmid.

13. Vector of claims 10 to 12, wherein said S-adenosyl-methionine synthetase is from the rat.

14. Vector of claim 13, which is contained in the *Escherichis coli* strain deposited as DSM 8592.

15. Bacterial host, characterized in that it is transformed with a vector according to claims 10 to 14.

16. Bacterial host of claim 15, which is *Escherichia coli.*

17. Bacterial host of claim 16, wherein said *Escherichia coli* is the *Escherichia coli* strain deposited as DSM 8592.

18. Method of production of a bacterial strain suitable for production of S-adenosyl-methionine, characterized in that a bacterium is transformed with an expression vector, said expression vector containing a nucleic acid sequence coding for S-adenosyl-methionine synthetase.
